(19) Europäisches Patentamt
European Patent Office
Office européen des brevets

(11) Numéro de publication: **0 269 841 B1**

(12) # FASCICULE DE BREVET EUROPEEN

(45) Date de publication de fascicule du brevet: **11.11.92**

(51) Int. Cl.[5]: **C07D 473/04**, A61K 31/52

(21) Numéro de dépôt: **87115591.7**

(22) Date de dépôt: **23.10.87**

Le dossier contient des informations techniques présentées postérieurement au dépôt de la demande et ne figurant pas dans le présent fascicule.

(54) **1-Hydroxyalkylxanthines, leurs procédés de préparation et médicament les contenant.**

(30) Priorité: **27.10.86 CH 4253/86**

(43) Date de publication de la demande:
**08.06.88 Bulletin 88/23**

(45) Mention de la délivrance du brevet:
**11.11.92 Bulletin 92/46**

(84) Etats contractants désignés:
**AT BE CH DE ES FR GB GR IT LI LU NL SE**

(56) Documents cités:
**FR-A- 1 211 333**
**US-A- 2 517 410**

**E. Schröder, Pharmazeutische Chemie, Ed. Georg Thieme Stuttgart-New York, 1982, page 702**

**The Pharmacological Basis of Therapeutics, Ed. H.S. Gilman, Macmillan, New York, 1980, pages 592-607**

**Cardiovascular Pharmacology, 2ème Ed., Raven Press, 1984, pages 314-321**

(73) Titulaire: **SOCIETE DES PRODUITS NESTLE S.A.**
**Case postale 353**
**CH-1800 Vevey(CH)**

(72) Inventeur: **Fumeaux, René**
**Avenue des Alpes 66**
**CH-1814 La Tour-de-Peilz(CH)**
Inventeur: **Philipposian, Georges**
**Avenue Valmont 18**
**CH-1010 Lausanne(CH)**

**EP 0 269 841 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne une nouvelle classe de xanthines, à savoir des 1-hydroxyalkylxanthines, ainsi que leurs procédés de préparation et un médicament les contenant.

Le brevet US-A-2 597 410 concerne des hydroxyalkylxanthines qui possèdent au moins un azote non-substitué.

Le brevet EP 39780 concerne des xanthines 1,3 ou 1,3,8 substituées présentant des propriétés sédatives et anxiolytiques. Ces composés ont été développés dans le but de renforcer ou de rendre plus spécifiques certains effets physiologiques d'intérêt thérapeutique des xanthines naturelles comme la caféine ou la théophylline. Malgré leur indéniable caractère neuroleptique et anxiolytique, les composés, objet du brevet précité, présentent des effets secondaires, notamment sur la fonction cardiovasculaire. Par contre, considérée isolément, l'activité cardio-stimulante de ces composés ouvrait une voie d'investigation dans cette direction.

La présente invention concerne une nouvelle classe de xanthines, à savoir des 1-hydroxyalkylxanthines, ayant notamment une activité cardiovasculaire. Les composés selon l'invention sont des xanthines de formule générale

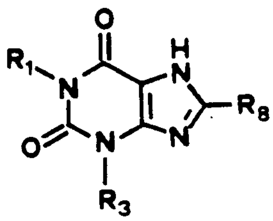

(I)

ou leurs sels physiologiquement acceptables, formule dans laquelle $R_1$ est un groupe $\omega$-hydroxy-n-alkyle en $C_2$-$C_5$ ou ($\omega$-1)-hydroxy-n-alkyle en $C_3$-$C_5$, $R_3$ est un groupe alkyle en $C_1$-$C_4$, $R_8$ est H, méthyle ou éthyle et la somme des atomes de carbone sur $R_1$ et $R_3$ est comprise entre 4 et 9.

De manière plus précise, les composés suivants sont plus spécifiquement revendiqués :

1-(2-Hydroxyéthyl)-3-propylxanthine,
1-(2-Hydroxyéthyl)-3-isobutylxanthine,
1-(2-Hydroxyéthyl)-3-isobutyl-8-méthylxanthine,
1-(2-Hydroxypropyl)-3-propylxanthine,
1-(2-Hydroxypropyl)-3-propyl-8-méthylxanthine,
1-(2-Hydroxypropyl)-3-butylxanthine,
1-(3-Hydroxypropyl)-3-propylxanthine,
1-(3-Hydroxypropyl)-3-propyl-8-méthylxanthine,
1-(3-Hydroxypropyl)-3-propyl-8-éthylxanthine,
1-(3-Hydroxypropyl)-3-butylxanthine,
1-(3-Hydroxypropyl)-3-isobutylxanthine,
1-(3-Hydroxypropyl)-3-isobutyl-8-méthylxanthine,
1-(3-Hydroxybutyl)-3-méthylxanthine,
1-(3-Hydroxybutyl)-3-éthylxanthine,
1-(3-Hydroxybutyl)-3-éthyl-8-méthylxanthine,
1-(3-Hydroxybutyl)-3-propylxanthine,
1-(3-Hydroxybutyl)-3-isobutylxanthine,
1-(4-Hydroxybutyl)-3-éthylxanthine,
1-(4-Hydroxybutyl)-3-propylxanthine,
1-(4-Hydroxybutyl)-3-propyl-8-méthylxanthine,
1-(4-Hydroxybutyl)-3-butylxanthine,
1-(4-Hydroxybutyl)-3-isobutyl-8-méthylxanthine,
1-(4-Hydroxypentyl)-3-méthylxanthine,
1-(4-Hydroxypentyl)-3-propylxanthine,
1-(5-Hydroxypentyl)-3-méthylxanthine,
1-(5-Hydroxypentyl)-3-propylxanthine et
1-(5-Hydroxypentyl)-3-propyl-8-méthylxanthine.

Par sels physiologiquement acceptables des composés de formule générale (I), on entend les sels que

forment ces composés avec des bases pharmaceutiquement acceptables : il s'agit de sels dont les cations présentent une inocuité vis-à-vis des organismes animaux et ne provoquent pas d'effet secondaire propre aux doses thérapeutiques. On peut citer les sels de métaux alcalins tels que le sodium, le potassium, les sels d'ammonium et d'amines pharmaceutiquement acceptables connues de spécialiste. Ces sels sont préparés par chauffage du composé de formule générale (I) en présence de base appropriée avec ou sans solvant, de préférence suivi d'une recristallisation.

Les composés selon l'invention sont préparés selon l'un des procédés ci-après :

A) on fait réagir un uracile de formule

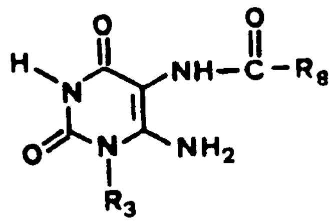

(II)

avec un agent alkylant de formule $R_1$-X où $R_1$, $R_3$ et $R_8$ sont définis dans la formule générale (I), sauf que $R_1$ est différent de ω-hydroxybutyle et où X est un halogène, de préférence le brome, ou mono- ou disulfate ou p-toluène sulfonate dans un solvant mutuel des reactifs tels que par exemple la diméthylformamide (DMF), le diméthylsulfoxyde (DMSO) ou l'hexaméthylphosphorotriamide (HMPT) à une température comprise entre 20 et 40 °C et en présence d'un hydroxyde alcalin, par exemple l'hydroxyde de sodium sous forme solide. La réaction est conduite de préférence dans la DMF à 20 °C selon le schéma réactionnel ci-après.

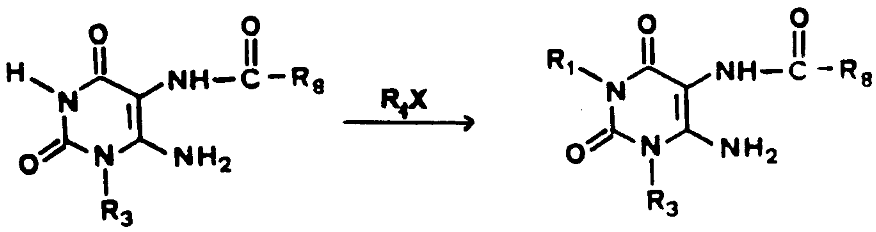


(III)

On opère ensuite la cyclisation du produit de formule générale (III) dans une solution d'hydroxyde alcalin entre 20 et 100 °C selon le schéma réactionnel ci-dessous :

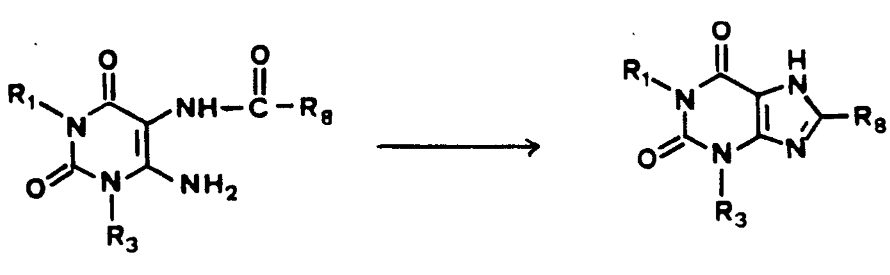

(I)

Bien qu'il soit possible d'isoler le dérivé de formule générale (III), on préfère opérer directement la cyclisation sans isoler ou purifier celui-ci. A cet effet, on neutralise le milieu réactionnel et on évapore le solvant, puis on dissout le résidu dans une solution d'hydroxyde alcalin et on chauffe au reflux.

B) Selon une variante, on effectue un alkylation de l'uracile (II) avec un agent contenant une fonction transformable en groupe hydroxyle, par exemple une fonction -COO-$R_4$ selon le schéma réactionnel suivant :

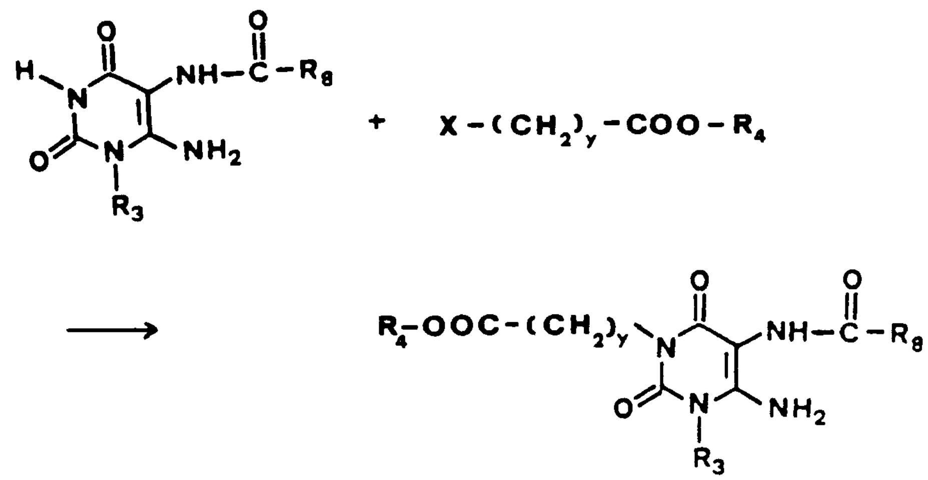

(IV)

dans lequel $R_3$, $R_8$ et X sont comme définis ci-dessus, y est compris entri 1 et 4 et $R_4$ est un groupe alkyle. Cette réaction d'alkylation se fait comme pour A) dans un solvant mutel des réactifs tels que la DMF, le DMSO ou le HMPT à une température comprise entre 20 et 40 ° C et en présence d'un hydroxyde alcalin sous forme solide. On effectue ensuite une cyclisation et une hydrolyse concomittante de l'ester (IV) dans une solution d'hydroxyde alcalin entre 20 et 100 ° C selon le schéma réactionnel suivant :

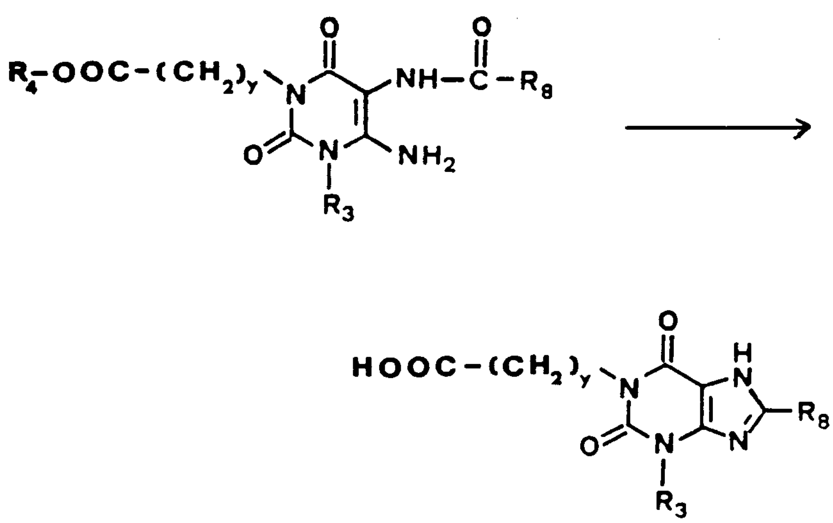

(V)

Bien qu'on puisse directement réduire l'acide (V) en alcool, on préfère réestérifier l'acide (V) avec un alcool tel que le méthanol, l'éthanol ou le propanol sous reflux pour obtenir l'ester correspondant qu'on réduit de manière connue, par exemple en présence de $LiAlH_4$ dans du THF pour obtenir le produit selon l'invention suivant :

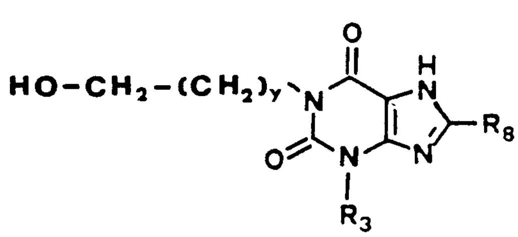

(I)

On voit donc que selon cette seconde forme du procédé selon l'invention, on obtient toujours des ω-hydroxyalkylxanthines.

C) Selon un dernier mode de mise en oeuvre du procédé selon l'invention, on prépare des (ω-1)-hydroxyalkylxanthines en alkylant l'uracile de formule générale

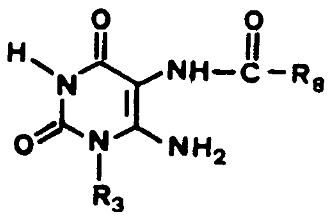

(II)

avec un agent alkylant de formule $R_5X$, où $R_3$, $R_8$ et X sont comme définis ci-dessus et $R_5$ est un groupe (ω-1)-alcényle en $C_3$-$C_5$. L'alkylation se fait dans les mêmes conditions que pour A) et B), à savoir dans un solvant mutuel des réactifs tels que la DMF, le DMSO ou le HMPT à une température comprise entre 20 et 40 ° C et en présence d'un hydroxyde alcalin sous forme solide. On effectue ensuite un cyclisation dans une solution d'hydroxyde alcalin entre 20 et 100 ° C selon le schéma réactionnel suivant :

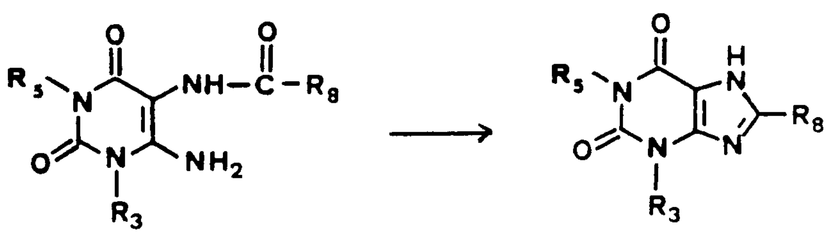

(VI)

Il reste finalement à hydrater la double liaison de $R_5$ selon une addition de type Markownikoff. Cette addition est réalisée suivant deux variantes :

Variante A : au moyen d'acide sulfurique dilué à une température d'environ 100 ° C pendant plusieurs jours.

Variante B : par la méthode d'oxymercuration-démercuration réductive en présence d'acétate de mercure puis de $NaBH_4$. (Larock, R.C. : Solvomercuration/Demercuration Reactions in Organic Synthesis, Springer Verlag, Berlin, 1986, Chap. 2).

L'invention concerne également une composition pharmaceutique contenant un composé de formule générale (I) en combinaison avec un support inerte pharmaceutiquement acceptable.

Le médicament selon l'invention se présente sous diverses formes pharmaceutiques contenant les excipients ou véhicules habituels tels que les comprimés, capsules, suppositoires, solutions, suspensions et être administrés par voie orale, sublinguale, rectale, sous-cutanée, intramusculaire, intraveineuse ou par inhalation.

Avant de donner les exemples proprement dits de synthèse des composés selon l'invention, le Tableau I ci-dessous donne pour 27 composés selon l'invention, le mode de préparation et l'agent alkylant à utiliser. Le Tableau II donne pour ces mêmes 27 composés le point de fusion et le solvant de recristallisation et le tableau III les valeurs de RMN du carbone 13.

TABLEAU I

| Xanthine No. | Composé selon l'invention | Préparation | Agent alkylant |
|---|---|---|---|
| 1 | 1-(2-Hydroxyethyl)-3-propylxanthine | Méthode A | 2-Bromethanol |
| 2 | 1-(2-Hydroxyethyl)-3-isobutylxanthine | Méthode A | 2-Bromethanol |
| 3 | 1-(2-Hydroxyethyl)-3-isobutyl-8-methylxanthine | Méthode A | 2-Bromethanol |
| 4 | 1-(2-Hydroxypropyl)-3-propylxanthine | Méthode C/Variante B | Bromure d'allyle |
| 5 | 1-(2-Hydroxypropyl)-3-propyl-8-methylxanthine | Méthode C/Variante A | Bromure d'allyle |
| 6 | 1-(2-Hydroxypropyl)-3-butylxanthine | Méthode C/Variante B | Bromure d'allyle |
| 7 | 1-(3-Hydroxypropyl)-3-propylxanthine | Méthode A | 3-Bromo-1-propanol |
| 8 | 1-(3-Hydroxyproply)-3-propyl-8-methylxanthine | Méthode A | 3-Bromo-1-propanol |
| 9 | 1-(3-Hydroxypropyl)-3-propyl-8-ethylxanthine | Méthode A | 3-Bromo-1-propanol |
| 10 | 1-(3-Hydroxypropyl)-3-butylxanthine | Méthode A | 3-Bromo-1-propanol |
| 11 | 1-(3-Hydroxypropyl)-3-isobutylxanthine | Méthode A | 3-Bromo-1-propanol |
| 12 | 1-(3-Hydroxypropyl)-3-isobutyl-8-methylxanthine | Méthode A | 3-Bromo-1-propanol |
| 13 | 1-(3-Hydroxybutyl)-3-methylxanthine | Méthode C/Variante A | 4-Bromo-1-butène |
| 14 | 1-(3-Hydroxybutyl)-3-ethylxanthine | Méthode C/Variante A | 4-Bromo-1-butène |
| 15 | 1-(3-Hydroxybutyl)-3-ethyl-8-methylxanthine | Méthode C/Variante A | 4-Bromo-1-butène |
| 16 | 1-(3-Hydroxybutyl)-3-propylxanthine | Méthode C/Variante A | 4-Bromo-1-butène |
| 17 | 1-(3-Hydroxybutyl)-3-isobutylxanthine | Méthode C/Variante A | 4-Bromo-1-butène |
| 18 | 1-(4-Hydroxybutyl)-3-ethylxanthine | Méthode B | 4-Bromobutyrate d'éthyle |
| 19 | 1-(4-Hydroxybutyl)-3-propylxanthine | Méthode B | 4-Bromobutyrate d'éthyle |
| 20 | 1-(4-Hydroxybutyl)-3-propyl-8-methylxanthine | Méthode B | 4-Bromobutyrate d'éthyle |
| 21 | 1-(4-Hydroxybutyl)-3-butylxanthine | Méthode B | 4-Bromobutyrate d'éthyle |
| 22 | 1-(4-Hydroxybutyl)-3-isobutyl-8-methylxanthine | Méthode B | 4-Bromobutyrate d'éthyle |
| 23 | 1-(4-Hydroxypentyl)-3-methylxanthine | Méthode C/Variante A | 5-Bromo-1-pentène |
| 24 | 1-(4-Hydroxypentyl)-3-propylxanthine | Méthode C/Variante B | 5-Bromo-1-pentène |
| 25 | 1-(5-Hydroxypentyl)-3-methylxanthine | Méthode A | 5-Bromo-1-pentanol |
| 26 | 1-(5-Hydroxypentyl)-3-propylxanthine | Méthode A | 5-Bromo-1-pentanol |
| 27 | 1-(5-Hydroxypentyl)-3-propyl-8-methylxanthine | Méthode A | 5-Bromo-1-pentanol |

TABLEAU II

| Xanthine No. | PF (°C) | Solvant recristallisation |
|---|---|---|
| 1 | 178-179 | Acétone |
| 2 | 200-202 | Acétone |
| 3 | 227-229 | Chloroforme |
| 4 | 150-152 | Chloroforme |
| 5 | 196-197 | Méthanol |
| 6 | 157-158 | Eau |
| 7 | 145-146 | Méthanol |
| 8 | 221-222 | Méthanol |
| 9 | 196-198 | Eau |
| 10 | 111-112 | Eau |
| 11 | 159-160 | Acétone |
| 12 | 248-250 | Ethanol |
| 13 | 200-201 | Acétone |
| 14 | 217-218 | Eau |
| 15 | 212-213 | Eau |
| 16 | 163-164 | Acétone |
| 17 | 141-142 | Acétone |
| 18 | 207-208 | Eau |
| 19 | 173-174 | Acétone |
| 20 | 192-193 | Eau |
| 21 | 122-123 | Acétone |
| 22 | 211-212 | Méthanol |
| 23 | 180-181 | Acétone |
| 24 | 153-154 | Acétone |
| 25 | 190-191 | Eau |
| 26 | 151-152 | Eau |
| 27 | 184-185 | Dioxanne |

TABLEAU III : Valeurs RMN du carbone-13 (δ ppm ; solvant : DMSO-$d_6$ ; Température : ambiante, sauf *: 80 °C)

| Xanthine No. | Noyau purine C-2 | C-4 | C-5 | C-6 | C-8 | Substituants R-1 | R-3 | R-8 | |
|---|---|---|---|---|---|---|---|---|---|
| 1 | 150.8 | 147,7 | 106,5 | 154,4 | 140,4 | 42,6 57,9 | 44,4 20,8 10,9 | - | |
| 2 | 151,0 | 147,9 | 106,5 | 154,4 | 140,3 | 42,7 57,9 | 49,9 26,9 19,8(2x) | - | |
| 3 | 151.0 | 148,2 | 106,1 | 153,8 | 150,3 | 42,5 57,9 | 49,8 26,8 19,7(2x) | 14,2 | |
| 4 | 151,0 | 147,7 | 106,6 | 154,6 | 140,5 | 47,5 63,7 20,9 | 44,4 20,9 10,9 | - | |
| 5 | 151,0 | 147,9 | 106,2 | 154,0 | 150,4 | 47,3 63,6 20,9 | 44,4 20,9 10,9 | 14,2 | |
| 6 | 151,0 | 147,6 | 106,6 | 154,5 | 140,4 | 47,5 63,7 20,9 | 42,6 29,6 19,3 13.5 | - | |
| 7 | 150,6 | 147,6 | 106,5 | 154,2 | 140,5 | 38,4 31,0 58,8 | 44,4 20,8 10,9 | - | |
| 8 | 150,7 | 147,9 | 106,2 | 153,8 | 150,5 | 38,3 31,1 58,9 | 44,4 20,9 10,9 | 14,2 | |
| 9 | 150,7 | 147,8 | 106,2 | 153,9 | 155,4 | 38,3 31,1 58,9 | 44,3 20,8 10,9 | 21,7 12,2 | |
| 10 | 150,7 | 147,6 | 106,6 | 154,3 | 140,5 | 38,4 31,1 58,9 | 42,6 29,6 19,4 13.5 | - | |
| 11 | 150,8 | 147,7 | 106,3 | 154,1 | 139,9 | 38,1 30,9 58,7 | 49,7 26,6 19,4(2x) | - | |
| 12 | 150,8 | 148,0 | 106,0 | 153,6 | 150,0 | 37,9 30,9 58,6 | 49,5 26,5 19,3(2x) | 13,8 | * |
| 13 | 150,9 | 147,8 | 106,5 | 154,3 | 140.4 | 38,4 37,2 64,3 23,5 | 29,6 | - | |
| 14 | 150,4 | 147,3 | 106,6 | 154,3 | 140,6 | 38,3 37,2 64,3 23,5 | 38,0 13,1 | - | |
| 15 | 150,4 | 147,6 | 106,3 | 153,7 | 150,5 | 38,4 37,2 64,3 23,4 | 38,0 13,1 | 14,2 | |
| 16 | 150,7 | 147,6 | 106,3 | 154,3 | 140,5 | 38,3 37,2 64,4 23,4 | 44,4 20,8 10,9 | - | |
| 17 | 150,8 | 147,9 | 106,4 | 154,2 | 140,3 | 38,4 37,2 64,3 23,4 | 49,8 26,8 19,7(2x) | - | |
| 18 | 150,3 | 147,1 | 106,4 | 154,0 | 140,0 | 40,5 24,2 29,7 60,4 | 37,7 12,7 | - | |
| 19 | 150,6 | 147,6 | 106,5 | 154,3 | 140,5 | 40,5 24,4 30,0 60,6 | 44,4 20,8 10,9 | - | |
| 20 | 150,5 | 147,9 | 106,2 | 153,8 | 150,5 | 40,5 24,5 30,0 60,6 | 44,3 20,9 10,9 | 14,2 | |
| 21 | 150,6 | 147,7 | 106,6 | 154,3 | 140,5 | 40,6 24,5 30,0 60,7 | 42,6 29,7 19,4 13.5 | - | |
| 22 | 150,8 | 148,0 | 106,0 | 153,6 | 150,0 | 40,2 24,2 29,7 60,5 | 49,6 26,5 19,4(2x) | 13,8 | * |
| 23 | 150,9 | 147,9 | 106,4 | 154,2 | 140,4 | 40,8 24,2 36,2 65,7 23,5 | 29,6 | - | |
| 24 | 150,6 | 147,6 | 106,5 | 154,2 | 140,4 | 40,6 24,1 36,2 65,7 23,5 | 44,4 20,8 10,9 | - | |
| 25 | 150,9 | 147,8 | 106,5 | 154,2 | 140,3 | 40.6 27,4 22,9 32,2 60,6 | 29,6 | - | |
| 26 | 150.6 | 147,6 | 106,3 | 154,2 | 140,4 | 40,6 27,4 22,9 32,1 60,5 | 44,4 20,8 10,9 | - | |
| 27 | 150,5 | 147,9 | 106,1 | 153,7 | 150,5 | 40,5 27,4 22,9 32,2 60,5 | 44,3 20,8 10,9 | 14,2 | |

Les exemples suivants illustrent l'invention; dans ceux-ci les quantités sont pondérales sauf indication contraire.

Exemple 1

1-(5-Hydroxypentyl)-3-propyl-8-méthylxanthine (Méthode A, Xanthine No. 27)

On met en suspension, sous atmosphère d'azote, 22,6 g (0,1 mole) de 1-propyl-5-acétylamino-6-aminouracile (II) ($R_3$ = Méthyle, $R_8$ = Méthyle) dans 200 ml de diméthylformamide (DMF) anhydre. On ajoute 21,7 g (0,13 mole) de 5-bromo-1-penthanol, puis, sous agitation, on ajoute par portions de 1 g à la fois à intervalles de 1 heure 6 g (0,15 mole) de NaOH solide pulvérulent. Après la troisième addition, la suspension est complètement dissoute. On laisse reposer une nuit pour compléter la réaction. On évapore le solvant à l'évaporateur rotatif sous 0,1 mm Hg et à 40 ° C. Le résidu huileux, qui est l'uracile (III) ($R_1$ = 5-Hydroxypentyle, $R_3$ = Propyle, $R_8$-Méthyle), qu'on ne purifie pas, est dissous dans 100 ml de NaOH à 10% et la solution chauffée au reflux pendant 2 heures. On refroidit dans un bain de glace et on neutralise la solution à pH 5 avec de l'acide acétique. On filtre le produit précipité, on le lave à l'eau froide, et on le sèche à l'étuve vide. On dissout dans 500 ml d'éthanol et on décolore la solution au charbon actif pendant 1 nuit. On précipite le produit par addition de 1 l d'eau, on filtre et on répète le traitement au charbon actif. Le produit séché est recristallisé 2 fois dans 120 ml de dioxanne. Rendement : 23.5 g (80%); cristaux incolores; PF 184-185 ° C.

Exemple 2

1-(4-Hydroxybutyl)-3-butylxanthine (Méthode B, Xanthine No. 21)

On met en solution, sous atmosphère d'azote, 11,3 g (0,05 mole) de 1-butyl-5-formylamino-6-aminouracile (II) ($R_1$ = Butyle, $R_8$ = H) dans 200 ml de DMF. On ajoute 10,6 ml (0,075 mole) de 4-bromobutyrate d'éthyle, puis sous bonne agitation 2 g (0,05 mole) de NaOH solide pulvérulent par portions de 0,5 g chaque heure. L'addition terminée, on laisse réagir encore une nuit. On évapore le solvant et on dissout le résidu huileux de (IV) (substituant en position 1 = $(CH_2)_3COOEt$) dans 100 ml de NaOH 10%. On chauffe cette solution pendant 1/2 heure au reflux. On refroidit, on neutralise à pH 5 avec de l'acide acétique, on filtre et sèche le précipité formé. Rendement : 9,2 g (60%) de xanthine (V) brute (substituant en position 1 = $(CH_2)_3COOH$, $R_3$ = Butyle, $R_8$ = H).

Sans purifier, on estérifie le produit avec 200 ml de méthanol contenant 1 ml d'acide sulfurique concentré au reflux pendant 5 heures. On concentre la solution à petit volume et on ajoute 50 ml d'eau. Le précipité d'ester qui se forme (substituant en position 1 = $(CH_2)_3COOMe$) est filté, lavé et séché. Rendement : 9 g (93%).

Sans purifier, on dissout ce produit dans 200 ml de tétrahydrofurane (THF) anhydre, on refroidit entre 0 et -10 ° C et on ajoute goutte à goutte une solution contenant 2,13 g (0,056 mole) de $LiAlH_4$ dans 150 ml de THF anhydre. L"addition terminée, on laisse encore réagir 2 heures à 0 ° C, puis on détruit l'excès de $LiAlH_4$ par addition de 50 ml d'eau. On acidifie le mélange à pH 2 avec du HCl concentré, puis on évapore le THF. La solution aqueuse résiduelle est extraite en continu dans du dichlorométhane une nuit. On sèche l'extrait organique et on évapore le solvant. Le résidu solide légèrement coloré (6,1 g) est recristallisé 2 fois dans l'acétone. Rendement : 4,6 g (33% à partir de II); cristaux incolores; PF 122-123 ° C.

Exemple 3

1-(3-Hydroxybutyl)-3-isobutylxanthine (Méthode C/Variante A, Xanthine No. 17)

On dissout 11,3 g (0,05 mole) de 1-isobutyl-5-formylamino-6-aminouracile (II) ($R_3$ = isobutyle, $R_8$ = H) dans 220 ml de DMF. On ajoute 8,8 g (0,065 mole ) de 4-bromo-1-butène, puis, sous agitation, 3 g (0,075 mole) de NaOH en poudre par portions de 0,5 g toutes les heures. On abandonne le mélange durant la nuit, puis on évapore le solvant sous vide. On reprend le résidu brut ($R_5$ = 3-butényle) dans 100 ml de NaOH 10% et on chauffe 1/2 heure à reflux. On refroidit la solution, on neutralise à pH 5 avec de l'acide acétique, on filtre le précipité formé et on le sèche. On obtient 8,5 g de produit solide brut (VI) ($R_5$ = 3-butényle, $R_3$ = isobutyle, $R_8$ = H). On purifie ce produit par passage sur une colonne de silice (85 g) en éluant avec du chloroforme : 6,5 g de produit incolore.

On chauffe à 100 ° C pendant 4 jours un mélange contenant 6 g du composé précédent dans 100 ml d'acide sulfurique 20%. On refroidit et neutralise à pH 5 avec du KOH 50%. On évapore la solution à sec, on reprend le résidu dans 100 ml d'éthanol absolu bouillant et on filtre l'insoluble. Le filtrat est débarassé du solvant laissant un résidu solide coloré (6,4 g), qu'on passe sur une colonne de silice (650 g) en éluant avec un gradient de chloroforme/méthanol (0-5% méthanol). La xanthine est recristallisée 2 fois dans l'acétone. Rendement : 4,5 g (33% à partir de II); PF = 141-142 ° C.

Exemple 4

1-(2-Hydroxypropyl)-3-butylxanthine (Méthode C/Variante B, Xanthine No. 6)

On dissout 16 g (0,050 mole d'acétate de mercure (2) dans 250 ml d'eau. Sous agitation, on ajoute goutte à goutte pendant 10 minutes 12,4 g (0,05 mole) de 1-allyl-3-butylxanthine (préparée comme ci-dessus à partir du 1-butyl-5-formyl-amino-6-aminouracile et de bromure d'allyle) dissoute dans 250 ml de THF. Au bout de quelques minutes, un précipité se forme. On poursuit l'agitation pendant 30 minutes, puis on refroidit le mélange dans un bain de glace. On ajoute 68 ml de NaOH 3-N, puis goutte à goutte, 60 ml d'une solution 0,5 M fraîchement préparée de $NaBH_4$ dans du NaOH 3-N. L'addition terminée, on poursuit l'agitation durant encore 15 min. On neutralise à pH 4-5 avec du HCl 6-N (env. 60 ml). On sature la solution avec du NaCl et on extrait la xanthine dans le dichlorméthane (3×200 ml). On sèche l'extrait organique et on évapore le solvant : 12,9 g de produit brut contenant 20% de produit de départ. On sépare les produits en passant le mélange sur une colonne de silice et en éluant avec du chloroforme de produit de départ, puis avec chloroforme/méthanol 95/5 la xanthine hydroxylée (9,5 g). On recristallise 2 fois dans l'eau : 8,5 g (64%); PF 157-158°C.

Exemple 5

Les composés (I) selon l'invention ont été trouvés peu toxiques et n'ont pas ou peu d'effet sur le système nerveux central. Par contre, ils se sont montrés actifs dans les test in vitro d'activité inotropique (cardiotonique). Le Tableau (IV) ci-dessous donne les résultats de ces essais.

TABLEAU (IV)

| Xanthine No. | Cardiotonique [1)] (concentration minimale efficace en ug/ml produisant une réponse significative) |
|---|---|
| 1 | 1 |
| 2 | 2,5 |
| 3 | 2 |
| 4 | 3,3 |
| 5 | 0,6 |
| 6 | 4,2 |
| 7 | 1,3 |
| 8 | 4,2 |
| 9 | 2 |
| 10 | 0,4 |
| 11 | 0,7 |
| 12 | 0,5 |
| 13 | 50 |
| 14 | 0,8 |
| 15 | 0,6 |
| 16 | 0,1 |
| 17 | 1,3 |
| 18 | 3,3 |
| 19 | 0,4 |
| 20 | 0,7 |
| 21 | 2 |
| 22 | 25 |
| 23 | 33,3 |
| 24 | 0,7 |
| 25 | 6,7 |
| 26 | 0,2 |
| 27 | 0,3 |

Références

| | |
|---|---|
| Theophylline | 15 |
| Amrinone [2)] | 100 |
| Milrinone [2)] | 0,5 |
| IBMX[3)] | 0,5 |

1) Mesure de la force de contraction de l'oreillette gauche du cobaye isolée et stimulée électriquement, selon Erjavek, F. and Adamic, S., Arch. Int. Pharmacodyn. 155: 251, 1965. Une réponse est considérée comme significative lorsque la force de contraction de base est augmentée de plus de 40 %.

2) Médicament cardiotonique selon l'état de la technique

3) 1-méthyl-3-isobutylxanthine

## Revendications

1. Composé de formule :

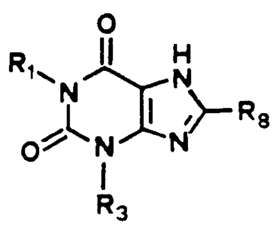

(I)

ou un sel physiologiquement acceptable de ce composé, formule dans laquelle $R_1$ est une groupe ω-hydroxy-n-alkyle en $C_2$-$C_5$ ou (ω-1)-hydroxy-n-alkyle en $C_3$-$C_5$, $R_3$ est un groupe alkyle en $C_1$-$C_4$, $R_8$ est H, méthyle ou éthyle et la somme des atomes de carbone sur $R_1$ et $R_3$ est comprise entre 4 et 9.

2. Composé selon la revendication 1, caractérisé en ce qu'il est choisi dans le groupe constitué par
1-(2-Hydroxyéthyl)-3-propylxanthine,
1-(2-Hydroxyéthyl)-3-isobutylxanthine,
1-(2-Hydroxyéthyl)-3-isobutyl-8-méthylxanthine,
1-(2-Hydroxypropyl)-3-propylxanthine,
1-(2-Hydroxypropyl)-3-propyl-8-méthylxanthine,
1-(2-Hydroxypropyl)-3-butylxanthine
1-(3-Hydroxypropyl)-3-propylxanthine,
1-(3-Hydroxypropyl)-3-propyl-8-méthylxanthine,
1-(3-Hydroxypropyl)-3-propyl-8-éthylxanthine,
1-(3-Hydroxypropyl)-3-butylxanthine,
1-(3-Hydroxypropyl)-3-isobutylxanthine,

1-(3-Hydroxypropyl)-3-isobutyl-8-méthylxanthine,
1-(3-Hydroxybutyl)-3-méthylxanthine,
1-(3-Hydroxybutyl)-3-éthylxanthine,
1-(3-Hydroxybutyl)-3-éthyl-8-méthylxanthine,
1-(3-Hydroxybutyl)-3-propylxanthine,
1-(3-Hydroxybutyl)-3-isobutylxanthine,
1-(4-Hydroxybutyl)-3-éthylxanthine,
1-(4-Hydroxybutyl)-3-propylxanthine,
1-(4-Hydroxybutyl)-3-propyl-8-méthylxanthine,
1-(4-Hydroxybutyl)-3-butylxanthine,
1-(4-Hydroxybutyl)-3-isobutyl-8-méthylxanthine,
1-(4-Hydroxypentyl)-3-méthylxanthine,
1-(4-Hydroxypentyl)-3-propylxanthine,
1-(5-Hydroxypentyl)-3-méthylxanthine,
1-(5-Hydroxypentyl)-3-propylxanthine,
1-(5-Hydroxypentyl)-3-propyl-8-méthylxanthine.

**3.** Procédé de préparation d'un composé de formule

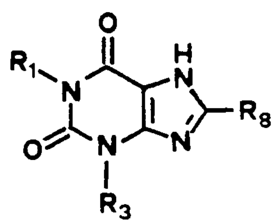

(I)

ou un sel physiologiquement acceptable de ce composé, formule dans laquelle $R_1$ est un groupe $\omega$-hydroxy-n-alkyle en $C_2$-$C_5$, ou ($\omega$-1) hydroxyle-n-alkyle en $C_3$-$C_5$, $R_3$ est un groupe alkyle en $C_1$-$C_4$, $R_8$ est H, méthyle ou éthyle et la somme des atomes de carbone sur $R_1$ et $R_3$ est comprise entre 4 et 9, caractérisé en ce que

A) on fait réagir un composé de formule

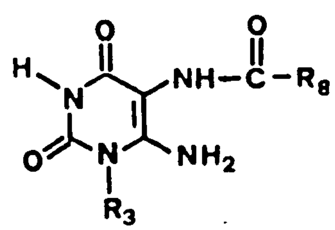

(II)

avec un agent alkylant de formule $R_1$-X où $R_1$, $R_3$ et $R_8$ sont comme définis ci-dessus sauf que $R_1$ est différent de $\omega$-hydroxybutyle et X est un halogène, mono- ou di-sulfate ou p-toluènesulfonate et on cyclise le composé obtenu dans une solution d'hydroxyde alcalin entre 20 et 100 ° C, ou

B) on fait réagir un composé de formule

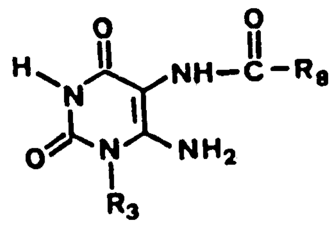

(II)

avec un agent alkylant de formule X-$(CH_2)_Y$-COO-$R_4$, où $R_3$, $R_8$ et X sont comme définis ci-dessus, Y est compris entre 1 et 4 et $R_4$ est un groupe alkyle, on cyclise en milieu alcalin aqueux le composé obtenu pour arriver au composé

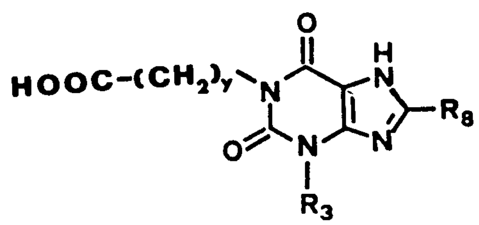

(V)

on estérifie (V) et on réduit l'ester obtenu pour arriver au composé (I) dans lequel $R_1$ est le groupe -$(CH_2)_Y$-$CH_2$-OH ou
C) on fait réagir un composé de formule

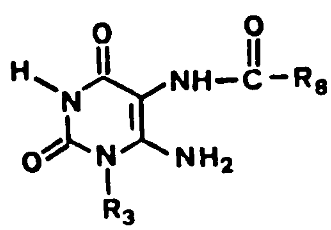

(II)

avec un agent alkylant de formule $R_5X$, où $R_3$, $R_8$ et X sont comme définis ci-dessus et $R_5$ est un groupe alcényle en $C_3$-$C_5$, on cyclise le composé obtenu dans une solution d'hydroxyde alcalin entre 20 et 100 °C puis on hydrate la double liaison de $R_5$ selon une addition de type Markownikoff pour obtenir (I) dans lequel $R_1$ est un groupe ($\omega$-1)-hydroxy-n-alkyle en $C_3$-$C_5$.

**4.** Composition pharmaceutique, caractérisée en ce qu'elle contient un composé de formule

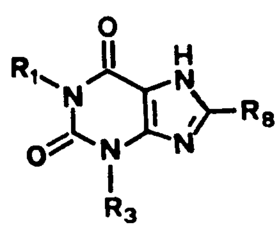

(I)

ou un sel physiologiquement acceptable de ce composé, formule dans laquelle $R_1$ est un groupe $\omega$-hydroxy-n-alkyle en $C_2$-$C_5$ ou ($\omega$-1)-hydroxy-n-alkyle en $C_3$-$C_5$, $R_3$ est un groupe alkyle en $C_1$-$C_4$, $R_8$ est H, méthyle ou éthyle et la somme des atomes de carbone sur $R_1$ et $R_3$ est comprise entre 4 et 9 en liaison avec un excipient pharmaceutiquement acceptable.

**5.** Composition pharmaceutique selon la revendication 4, caractérisée en ce qu'elle contient un composé

selon l'une des revendications 2 et 3.

**Claims**

1. A compound corresponding to the following general formula

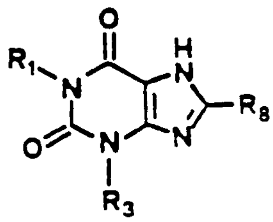

(I)

in which $R_1$ is a $C_{2-5}$ ω-hydroxy-n-alkyl group or a $C_{3-5}$ (ω-1)-hydroxy-n-alkyl group, $R_3$ is a $C_{1-4}$ alkyl group, $R_8$ is H, methyl or ethyl and the sum of the carbon atoms in $R_1$ and $R_3$ is between 4 and 9, or a physiologically acceptable salt of this compound.

2. A compound as claimed in claim 1, characterized in that it is selected from the group comprising:
1-(2-hydroxyethyl)-3-propyl xanthine,
1-(2-hydroxyethyl)-3-isobutyl xanthine,
1-(2-hydroxyethyl)-3-isobutyl-8-methyl xanthine,
1-(2-hydroxypropyl)-3-propyl xanthine,
1-(2-hydroxypropyl)-3-propyl-8-methyl xanthine,
1-(2-hydroxypropyl)-3-butyl xanthine,
1-(3-hydroxypropyl)-3-propyl xanthine,
1-(3-hydroxypropyl)-3-propyl-8-methyl xanthine,
1-(3-hydroxypropyl)-3-propyl-8-ethyl xanthine,
1-(3-hydroxypropyl)-3-butyl xanthine,
1-(3-hydroxypropyl)-3-isobutyl xanthine,
1-(3-hydroxypropyl)-3-isobutyl-8-methyl xanthine,
1-(3-hydroxybutyl)-3-methyl xanthine,
1-(3-hydroxybutyl)-3-ethyl xanthine,
1-(3-hydroxybutyl)-3-ethyl-8-methyl xanthine,
1-(3-hydroxybutyl)-3-propyl xanthine,
1-(3-hydroxybutyl)-3-isobutyl xanthine,
1-(4-hydroxbutyl)-3-ethyl xanthine,
1-(4-hydroxbutyl)-3-propyl xanthine,
1-(4-hydroxbutyl)-3-propyl-8-methyl xanthine,
1-(4-hydroxbutyl)-3-butyl xanthine,
1-(4-hydroxbutyl)-3-isobutyl-8-methyl xanthine,
1-(4-hydroxypentyl)-3-methyl xanthine,
1-(4-hydroxypentyl)-3-propyl xanthine,
1-(5-hydroxypentyl)-3-methyl xanthine,
1-(5-hydroxypentyl)-3-propyl xanthine and
1-(5-hydroxypentyl)-3-propyl-8-methyl xanthine.

3. A process for the preparation of a compound corresponding to the following formula

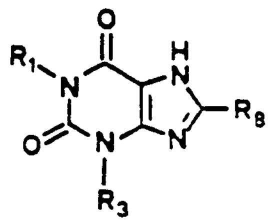

(I)

in which $R_1$ is a $C_{2-5}$ $\omega$-hydroxy-n-alkyl group or a $C_{3-5}$ ($\omega$-1)-hydroxy-n-alkyl group, $R_3$ is a $C_{1-4}$ alkyl group, $R_8$ is H, methyl or ethyl and the sum of the carbon atoms in $R_1$ and $R_3$ is between 4 and 9, or a physiologically acceptable salt of this compound, characterized in that

A) a compound corresponding to the following formula

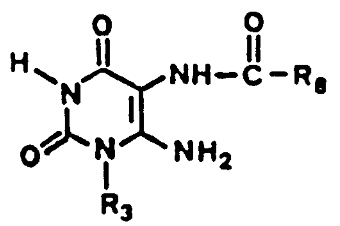

(II)

is reacted with an alkylating agent of the formula $R_1$-X, where $R_1$, $R_3$ and $R_8$ are as defined above, except that $R_1$ is not $\omega$-hydroxybutyl, and X is a halogen atom, monosulfate or disulfate or p-toluenesulfonate,

and the compound obtained is cyclized at 20 to 100 ° C in an alkali metal hydroxide solution, or

B) a compound corresponding to the following formula

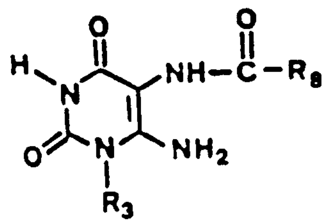

(II)

is reacted with an alkylating agent of the formula $X-(CH_2)_y-COO-R_4$, where $R_3$, $R_8$ and X are as defined above, y is a number of 1 to 4 and $R_4$ is an alkyl group,

the compound obtained is cyclized in aqueous alkaline medium to form the following compound

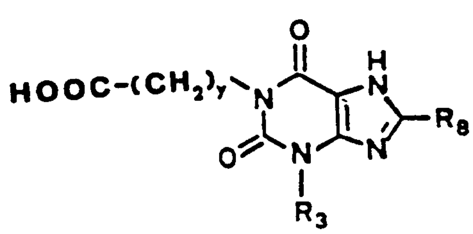

(V)

the compound (V) thus obtained is esterified and the ester obtained is reduced to form the compound (I), in which $R_1$ is the group $-(CH_2)_y-CH_2-OH$ or

C) a compound corresponding to the following formula

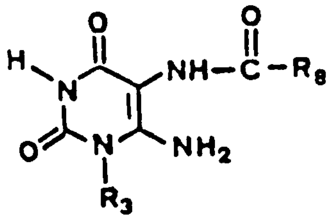

(II)

is reacted with an alkylating agent of the formula $R_5X$, where $R_3$, $R_8$ and X are as defined above and $R_5$ is a $C_{3-5}$ alkenyl group,
the compound obtained is cyclized at 20 to 100 ° C in an alkali metal hydroxide solution, the double bond of $R_5$ is then hydrated by an addition reaction of the Markownikoff type to form (I), in which $R_1$ is a C3-5 ($\omega$-1)-hydroxy-n-alkyl group.

**4.** A pharmaceutical composition, characterized in that it contains a compound corresponding to the following formula

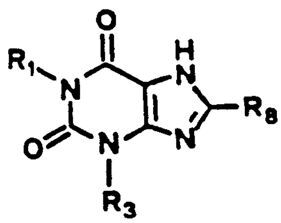

(I)

in which $R_1$ is a $C_{2-5}$ $\omega$-hydroxy-n-alkyl group or a $C_{3-5}$ ($\omega$-1)-hydroxy-n-alkyl group, $R_3$ is a $C_{1-4}$ alkyl group, $R_8$ is H, methyl or ethyl and the sum of the carbon atoms in $R_1$ and $R_3$ is between 4 and 9,
or a physiologically acceptable salt of this compound in conjunction with a pharmaceutically acceptable excipient.

**5.** A pharmaceutical composition as claimed in claim 4, characterized in that it contains a compound of the type claimed in claim 2 or 3.

**Patentansprüche**

**1.** Verbindung mit der Formel:

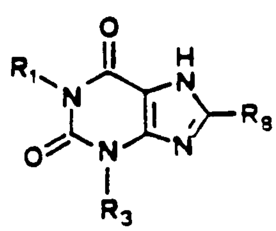

(I)

oder ein physiologisch annehmbares Salz dieser Verbindung, in welcher Formel $R_1$ eine $\omega$-Hydroxy-n-$C_2$-$C_5$-alkylgruppe oder ($\omega$-1)-Hydroxy-n-$C_3$-$C_5$-alkylgruppe ist, $R_3$ eine $C_1$-$C_4$-Alkylgruppe darstellt, $R_8$ für Wasserstoff, Methyl oder Ethyl steht und die Summe der Kohlenstoffatome in $R_1$ und $R_3$ von 4 bis 9 beträgt.

**2.** Verbindung nach Anspruch 1, dadurch gekennzeichnet, daß sie aus der aus
1-(2-Hydroxyethyl)-3-propylxanthin,
1-(2-Hydroxyethyl)-3-isobutylxanthin,
1-(2-Hydroxyethyl)-3-isobutyl-8-methylxanthin,

1-(2-Hydroxypropyl)-3-propylxanthin,
1-(2-Hydroxypropyl)-3-propyl-8-methylxanthin,
1-(2-Hydroxypropyl)-3-butylxanthin,
1-(3-Hydroxypropyl)-3-propylxanthin,
1-(3-Hydroxypropyl)-3-propyl-8-methylxanthin,
1-(3-Hydroxypropyl)-3-propyl-8-ethylxanthin,
1-(3-Hydroxypropyl)-3-butylxanthin,
1-(3-Hydroxypropyl)-3-isobutylxanthin,
1-(3-Hyroxypropyl)-3-isobutyl-8-methylxanthin,
1-(3-Hydroxybutyl)-3-methylxanthin,
1-(3-Hydroxybutyl)-3-ethylxanthin,
1-(3-Hydroxybutyl)-3-ethyl-8-methylxanthin,
1-(3-Hydroxybutyl)-3-propylxanthin,
1-(3-Hydroxybutyl)-3-isobutylxanthin,
1-(4-Hydroxybutyl)-3-ethylxanthin,
1-(4-Hydroxybutyl)-3-propylxanthin,
1-(4-Hydroxybutyl)-3-propyl-8-methylxanthin,
1-(4-Hydroxybutyl)-3-butylxanthin,
1-(4-Hydroxybutyl)-3-isobutyl-8-methylxanthin,
1-(4-Hydroxypentyl)-3-methylxanthin,
1-(4-Hydroxypentyl)-3-propylxanthin,
1-(5-Hydroxypentyl)-3-methylxanthin,
1-(5-Hydroxypentyl)-3-propylxanthin,
1-(5-Hydroxypentyl)-3-propyl-8-methylxanthin
bestehenden Gruppe ausgewählt ist.

**3.** Verfahren zur Herstellung einer Verbindung mit der Formel

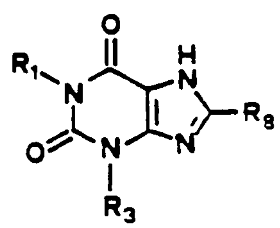

(I)

oder eines physiologisch annehmbares Salzes dieser Verbindung, in welcher Formel $R_1$ eine $\omega$-Hydroxy-n-$C_2$-$C_5$-alkylgruppe oder ($\omega$-1)-Hydroxy-n-$C_3$-$C_5$-alkylgruppe ist, $R_3$ eine $C_1$-$C_4$-Alkylgruppe darstellt, $R_8$ für Wasserstoff, Methyl oder Ethyl steht und die Summe der Kohlenstoffatome in $R_1$ und $R_3$ von 4 bis 9 beträgt, dadurch gekennzeichnet, daß man

A) eine Verbindung der Formel

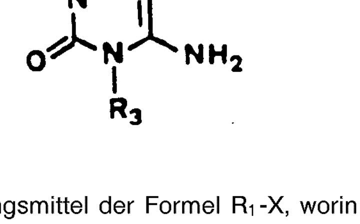

(II)

mit einem Alkylierungsmittel der Formel $R_1$-X, worin $R_1$, $R_3$ und $R_8$ wie oben definiert sind, mit der Ausnahme, daß $R_1$ von $\omega$-Hydroxybutyl verschieden ist, und worin X ein Halogen, Mono- oder Disulfat oder p-Toluolsulfonat ist, umsetzt und die erhaltene Verbindung in einer Alkalihydroxidlösung bei 20 bis 100 ° C cyclisiert, oder

B) eine Verbindung der Formel

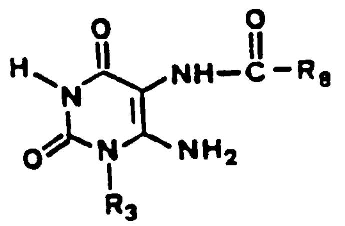

(II)

mit einem Alkylierungsmittel der Formel X-$(CH_2)_Y$-COO-$R_4$, worin $R_3$, $R_8$ und X wie vorstehend definiert sind, Y einen Wert von 1 bis 4 aufweist und $R_4$ eine Alkylgruppe bedeutet, umsetzt, die erhaltene Verbindung in wäßrig-alkalischem Milieu zu einer Verbindung

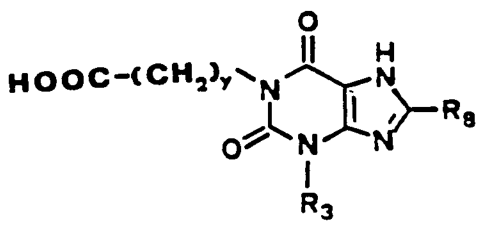

(V)

cyclisiert, die Verbindung (V) verestert und den erhaltenen Ester zu einer Verbindung (I) reduziert, worin $R_1$ die Gruppe -$(CH_2)_Y$-$CH_2$-OH darstellt, oder
C) eine Verbindung der Formel

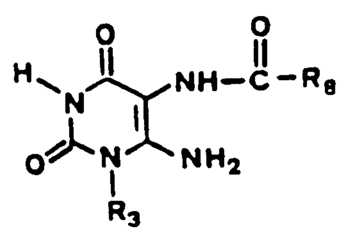

(II)

mit einem Alkylierungsmittel der Formel $R_5X$, worin $R_3$, $R_8$ und X wie vorstehend definiert sind und $R_5$ eine $C_3$-$C_5$-Alkenylgruppe bedeutet, umsetzt, die erhaltene Verbindung in einer Alkalihydroxidlösung bei 20 bis 100 °C cyclisiert und anschließend die Doppelbindung von $R_5$ nach einer Markownikoff-Addition hydratisiert, um eine Verbindung (I) zu erhalten, worin $R_1$ eine ($\omega$-1)-Hydroxy-n-$C_3$-$C_5$-alkylgruppe bedeutet.

**4.** Pharmazeutische Zusammensetzung, dadurch gekennzeichnet, daß sie eine Verbindung der Formel

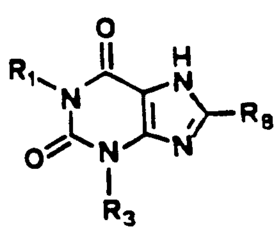

(I)

oder ein physiologisch annehmbares Salz dieser Verbindung, in welcher Formel $R_1$ eine $\omega$-Hydroxy-n-$C_2$-$C_5$-alkylgruppe oder ($\omega$-1)-Hydroxy-n-$C_3$-$C_5$-alkylgruppe ist, $R_3$ eine $C_1$-$C_4$-Alkylgruppe darstellt, $R_8$ für Wasserstoff, Methyl oder Ethyl steht und die Summe der Kohlenstoffatome in $R_1$ und $R_3$ von 4 bis

9 beträgt, in Verbindung mit einem pharmazeutisch annehmbaren Exzipiens enthält.

**5.** Pharmazeutische Zusammensetzung nach Anspruch 4, dadurch gekennzeichnet, daß sie eine Verbindung nach einem der Ansprüche 2 und 3 enthält.